# EUROPÄISCHE PATENTANMELDUNG

(11) **EP 0 872 471 A1**
(43) Veröffentlichungstag der Anmeldung: **21.10.1998**
(21) Anmeldenummer: 98810294.3
(22) Anmeldetag: 07.04.1998
(51) Int. Cl.: C07C 67/04, C07C 69/40

(54) **Verfahren zur Herstellung von Bernsteinsäuredi-tert.-butylester**

(30) Priorität: 15.04.1997 CH 873/97
(71) Anmelder: Ciba Specialty Chemicals Holding Inc., 4057 Basel (CH)
(72) Erfinder: Kudschus, Martin, 1762 Givisiez (CH); Wallquist, Olof, 1723 Marly (CH)

(57) **Zusammenfassung**

Verfahren zur Herstellung von Bernsteinsäure-di-tert.-butylester der Formel 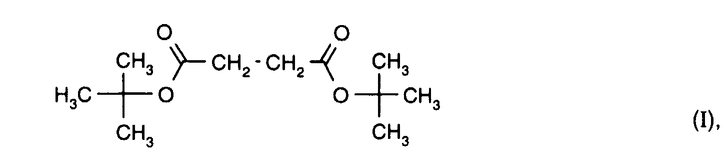 durch protonenkatalysierte Veresterung von Bernsteinsäure mit Isobuten, dadurch gekennzeichnet, dass man man Bernsteinsäure oder Bernsteinsäureanhydrid oder Mischungen davon mit Isobuten in Gegenwart eines wasserhaltigen, sauren Veresterungskatalysators, umsetzt, mit der Massgabe, dass man keine Schwefelsäure in einer Konzentration höher als 95 Gew.% einsetzt.

Dieses Verfahren erlaubt die Herstellung von Bernsteinsäuredi-tert.-butylester durch Umsetzung von Bernsteinsäure oder Bernsteinsäureanhydrid oder entsprechenden Mischungen aus Bernsteinsäure und Bernsteinsäureanhydrid mit Isobuten unter verminderter Wärmeentwicklung und gleichzeitiger Erhöhung der Ausbeute sowie gleichzeitiger verbesserter Reinheit des Endprodukts.

## Beschreibung

Die vorliegende Erfindung betrifft ein verbessertes Verfahren zur Herstellung von Bernsteinsäure-di-tert.-butylester der Formel 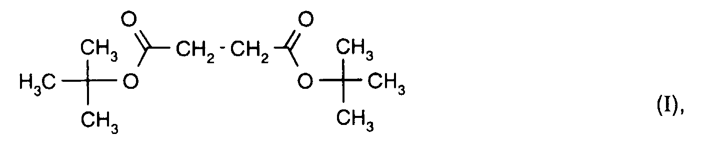 durch protonenkatalysierte Veresterung von Bernsteinsäure mit Isobuten.

Die Herstellung von tert.-Alkylester aus Carbonsäuren mit Alkenen in Gegenwart einer konzentrierten Säure entweder mit oder ohne Zugabe von aprotischen organischen Cosolvenzien, wie z.B. Diethylether oder Dioxan (A. L. McCloskey und G. S. Fonken, Org. Synth. Coll. Vol. 4, 261 (1963) ist bekannt.

Desweiteren ist die Veresterung von Bernsteinsäure mit Isobuten in Gegenwart von konzentrierter Schwefelsäure und tert.-Butanol als protisches Cosolvens von H. Pielartzik, B. Irmisch-Piellartzik, T. Eicher in Houben-Weyl Bd. E5/1, 662ff (1985) beschrieben.

Nachteilig für den technischen Gebrauch dieses Verfahrens ist die starke Wärmeentwicklung während der Zugabe der konzentrierten Schwefelsäure, die insbesondere bei Herstellungen im Produktionsmassstab nur sehr schwer kontrolliert werden kann und neben sicherheitstechnischen Risiken zu größeren Ausbeute- und Qualitätsschwankungen führt. So bilden sich beispielsweise Polymerisationsprodukte des Isobutens, deren Abtrennung vom Bernsteinsäure-di-tert.-butylester schwierig und damit wirtschaftlich nachteilig ist. Zudem sind notwendige Auf- und Umarbeitungen zeit-, arbeits- und energieaufwendig. Präventive Massnahmen zur Kontrolle der freiwerdenden Wärme erfordern apparativ aufwendige Kühlsysteme und eine entsprechend langsame Zulaufgeschwindigkeit der Schwefelsäure. Die sich hieraus ergebende eine teuerere Infrastruktur, lange Reaktionszeit sowie die schlechte Reproduzierbarkeit des Verfahrens wirken sich wirtschaftlich nachteilig aus.

Der Erfindung lag daher die Aufgabe zugrunde, den geschilderten Mängeln abzuhelfen und ein robustes, stabiles Verfahren zu finden, das wirtschaftlich attraktiv ist.

Demgemäss betrifft die vorliegende Erfindung ein verbessertes Verfahren zur Herstellung von Bernsteinsäure-di-tert.-butylester der Formel 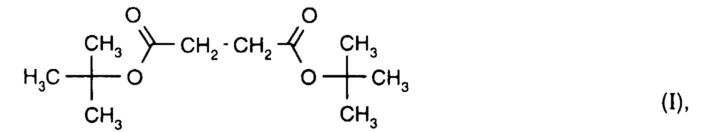 durch protonenkatalysierte Veresterung von Bernsteinsäure mit Isobuten, indem man Bernsteinsäure oder Bernsteinsäureanhydrid oder Mischungen davon mit Isobuten in Gegenwart eines wasserhaltigen, sauren Veresterungskatalysators, umsetzt, mit der Massgabe, dass man keine Schwefelsäure in einer Konzentration höher als 95 Gew.% einsetzt.

Das Molverhältnis von Bernsteinsäure oder Bernsteinsäureanhydrid oder Mischungen davon zu Isobuten wählt man in der Regel von 1:2 bis 1: 50, bevorzugt von 1:6 bis 1:13.

Das Molverhältnis von Bernsteinsäure zu Bernsteinsäureanhydrid in der Mischung wählt man in der Regel im Bereich von 1:100 bis 100:1, bevorzugt von 8:2 bis 2:8 und besonders bevorzugt beträgt es 1:1.

Erfindungsgemäss setzt man wasserhaltige, saure Veresterungskatalysatoren ein, mit der Massgabe, dass man keine Schwefelsäure in einer Konzentration höher als 95 Gew.% einsetzt. Üblicherweise verwendet man 40 bis 90, bevorzugt 40 bis 80 und besonders bevorzugt 50 bis 75 Gew. %-ige wässerige Mineralsäuren, wie Schwefelsäure, Phosphorsäure oder organische Sulfonsäuren, wie beispielsweise Methansulfonsäure.

Bei Verwendung von Bemsteinsäure setzt man bevorzugt, 65 bis 75 Gew. %-ige und bei Verwendung von Bernsteinsäureanhydrid 55 bis 65 Gew. %-ige wässerige Schwefelsäure.

Im allgemeinen setzt man pro Mol Bemsteinsäure oder Bernsteinsäureanhydrid oder einer Mischungen davon 0,1 bis 1,3 Mol Veresterungskatalysator ein.

Besonders bevorzugt werden auf ein Mol Bemsteinsäure oder Bernsteinsäureanhydrid 0,5 bis 0,8 Mol Schwefelsäure eingesetzt.

Die Veresterung wird in der Regel bei einer Temperatur im Bereich von 263 bis 333 K und einem Druck im Bereich von (1 × 10⁵) bis (25 × 10⁵) Pa durchgeführt. Bevorzugt beträgt die Reaktionstemperatur 283 bis 313 K bei (2 x 10⁵) bis (8 x 10⁵) Pa, ganz besonders bevorzugt 293 bis 303 K, wobei im letzteren Fall das Reaktionsgemisch bevorzugt unter einem Druck von (3 x 10⁵) bis (6 x 10⁵) Pa gehalten wird.

Bevorzugt führt man die Reaktion ohne organische Cosolvenzien oder Lösungsmitteln durch, man kann die Reaktion jedoch auch mit ihnen durchführen.

Die Wahl und Menge der organischen Lösungsmittel oder Cosolvenzien hängt vom bevorzugten Bereich ab und liegt in der Regel im Bereich von 0,01 bis 50 Gew.%, bevorzugt im Bereich von 0,01 bis 20 Gew.% und besonders bevorzugt im Bereich von 0,01 bis 10 Gew.% bezogen auf Isobuten.

Organische Lösungsmittel und Cosolvenzien bedeuten beispielsweise aprotische organische Lösungsmittel, wie beispielsweise ortho-Dichlorbenzol, oder aliphatische Kohlenwasserstoffe, wie beispielsweise Hexan, Octan, oder Ligroin, oder Ether, wie zum Beispiel Dioxan, Tetrahydrofuran oder Diethylether, oder protische Lösungsmittel, wie tert.-Alkohole, insbesondere tert.-Butanol oder tert.-Amylalkohol.

Die Vorteile des erfindungsgemässen Verfahrens liegen darin, dass die Veresterung bei bedeutend geringerer Wärmeentwicklung verläuft, und zugleich ein deutlich verunreinigungsärmeres Rohprodukt in hoher Ausbeute entsteht. Damit werden aufwendige Reinigungsverfahren, die für die Verwendung von Bernsteinsäure-di-tert.-butylester als ein Ausgangsprodukt zur Herstellung von 1,4-Diketopyrrolo-[3,4-c]-pyrrol-Pigmenten erforderlich sind, überflüssig.

Die nachfolgenden Beispiele erläutern die Erfindung:

### Beispiel 1 (wässerige Schwefelsäure / Edukt: Bernsteinsäure):

In einem Autoklaven werden 212,5 g (1,80 Mol) Bernsteinsäure vorgelegt und bei 263 K Manteltemperatur 935 g (16,65 Mol) Isobuten aufgepresst. Nach beendeter Zugabe werden innerhalb von 45 Minuten, bei 268 bis 275 K Innentemperatur, 146,4 g (1,08 Mol) Schwefelsäure, 72,3 Gew. % zugepumpt. Danach wird das Reaktionsgemisch 30 Minuten bei einer Innentemperatur von 275 bis 278 K und anschliessend, nach Temperaturerhöhung auf 293 bis 295 K für weitere 14 Stunden bei dieser Temperatur und einem Druck von (4 x 10⁵) bis (5 x 10⁵) Pa gerührt. Das Reaktionsgemisch drückt man auf eine gerührte Mischung bestehend aus 1000 g Eis und 300 ml (3 Mol) Natronlauge, 30 Gew. % ab und spült den Autoklaven anschließend mit 200 - 300 ml Petrolether, der ebenfalls zum Eis- / Natronlauge-Gemisch gegeben wird. Das Gemisch wird auf Raumtemperatur erwärmt und die organische Phase abgetrennt. Die wässerige Phase wird noch dreimal mit jeweils 100 - 150 ml Petrolether extrahiert und anschliessend die organischen Phasen vereinigt. Am Rotationsverdampfer werden die organischen Phasen bei einer Badtemperatur von 323 K und (3 × 10³) Pa (Endwert) eingeengt. Zur Reinigung des Rohproduktes wird dieses, unter Zusatz von ca. 0,5 g Magnesiumoxyd, über eine 30 cm Füllkörperkolonne (Raschigringe 5 x 5 mm) im Vakuum über Kopf destilliert. Man erhält 358,2 g (86,4 % der Theorie) Bernsteinsäure-di-tert.-butylester (Kp: 0,8 - 0,9 Pa bei 321 bis 324 K) mit einer gaschromatographisch ermittelten Reinheit von 99,0 % (Flächenprozent) entsprechend 85,6 % der Theorie à 100 %.
Dosierwärme: -26 KJ / Mol Schwefelsäure,
Totale Reaktionswärme: -108 KJ / Mol Bernsteinsäure.

### Beispiel 2 (Vergleich zum Stand der Technik in H. Pielartzik, B. Irmisch-Piellartzik, T. Eicher in Houben-Wevl Bd. E5/1. 662ff (1985), wo konzentrierte Schwefelsäure und tert.-Butanol als Cosolvens zur Veresteruna von Bernsteinsäure mit Isobuten verwendet werden):

In einem Autoklaven werden 212,5 g (1,80 Mol) Bemsteinsäure und 166,8 g (2,25 Mol) tert.-Butanol vorgelegt und bei 263 K Manteltemperatur 808 g (14,40 Mol) Isobuten aufgepresst. Nach beendeter Zugabe werden innerhalb von 70 Minuten, bei 268 bis 275 K Innentemperatur, 108,1 g (1,08 Mol) Schwefelsäure, 98 Gew. % zugepumpt. Danach wird das Reaktionsgemisch 30 Minuten bei einer Innentemperatur von 275 bis 278 K und anschliessend, nach Temperaturerhöhung auf 293 bis 295 K für weitere 14 Stunden bei dieser Temperatur und einem Druck von (4 x 10⁵) bis (5 x 10⁵) Pa gerührt. Das Reaktionsgemisch drückt man auf eine gerührte Mischung bestehend aus 1000 g Eis und 300 ml (3 Mol) Natronlauge, 30 Gew. % ab und spült den Autoklaven anschliessend mit 200 - 300 ml Petrolether, der ebenfalls zum Eis- / Natronlauge-Gemisch gegeben wird. Das Gemisch wird auf Raumtemperatur erwärmt und die organische Phase abgetrennt. Die wässerige Phase wird noch dreimal mit jeweils 100 - 150 ml Petrolether extrahiert und die organischen Phasen anschliessend vereinigt. Am Rotationsverdampfer werden die organischen Phasen bei einer Badtemperatur von 323 K und (3 × 10³) Pa (Endwert) eingeengt. Zur Reinigung des Rohproduktes wird dieses, unter Zusatz von ca. 0,5 g Magnesiumoxyd, über eine 30 cm Füllkörperkolonne (Raschigringe 5 x 5 mm) im Vakuum fraktioniert destilliert. Der Estergehalt der einzelnen Fraktionen wird gaschromatographisch ermittelt. Fraktionen mit Estergehalten < 97 Gew. % werden erneut destilliert. Man erhält, nach insgesamt drei Destillationen, 329,3 g (79,4 % der Theorie) Bernsteinsäure-di-tert.-butylester mit einer gaschromatographisch ermittelten Reinheit von 97,2 % (Flächenprozent) entsprechend 77,2 % der Theorie à 100 %.
Dosierwärme: -77 KJ / Mol Schwefelsäure,
Totale Reaktionswärme: - 120 KJ / Mol Bernsteinsäure.

### Beispiel 3 (Edukt: Bernsteinsäureanhydrid):

In einem Autoklaven werden 190,2 g (1,90 Mol) Bernsteinsäureanhydrid vorgelegt und bei 263 K Manteltemperatur 986 g (17,58 Mol) Isobuten aufgepresst. Nach beendeter Zugabe werden innerhalb von 45 Minuten, bei 266 bis 273 K Innentemperatur, 186,4 g (1,14 Mol) Schwefelsäure, 60,0 Gew. % zugepumpt. Danach wird das Reaktionsgemisch 30 Minuten bei einer Innentemperatur von 273 bis 278 K und anschliessend, nach Temperaturerhöhung auf 293 bis 295 K für weitere 14 Stunden bei dieser Temperatur und einem Druck von (4 x 10⁵) bis (5 x 10⁵) Pa gerührt. Das Reaktionsgemisch drückt man auf eine gerührte Mischung bestehend aus 1000 g Eis und 315 ml (3,15 Mol) Natronlauge, 30 Gew. % ab und spült den Autoklaven anschließend mit 200 - 300 ml Petrolether, der ebenfalls zum Eis- / Natronlauge-Gemisch gegeben wird. Das Gemisch wird auf Raumtemperatur erwärmt und die organische Phase abgetrennt. Die wässerige Phase wird noch dreimal mit jeweils 100 - 150 ml Petrolether extrahiert und die organischen Phasen anschliessend vereinigt. Am Rotationsverdampfer werden die organischen Phasen bei einer Badtemperatur von 323 K und (3 x 10³) Pa (Endwert) eingeengt. Zur Reinigung des Rohproduktes wird dieses, unter Zusatz von ca. 0,5 g Magnesiumoxyd, über eine 30 cm Füllkörperkolonne (Raschigringe 5 x 5 mm) im Vakuum über Kopf destilliert. Man erhält 385,0 g (88,0 % der Theorie) Bernsteinsäure-di-tert.-butylester (Kp: 0,4 - 0,6 Pa bei 320 bis 323 K) mit einer gaschromatographisch ermittelten Reinheit von 99,6 % (Flächenprozent) entsprechend 87,6 % der Theorie à 100 %.
Dosierwärme: -23 KJ / Mol Schwefelsäure,
Totale Reaktionswärme: -145 KJ / Mol Bernsteinsäureanhydrid.

### Beispiel 4 (erhöhte Reaktionstemperatur + verkürzte Reaktionsdauer):

In einem Autoklaven werden 212,5 g (1,80 Mol) Bernsteinsäure vorgelegt und bei 273 K Manteltemperatur 943 g (16,81 Mol) Isobuten aufgepresst. Nach beendeter Zugabe werden innerhalb von 45 Minuten, bei 275 bis 283 K Innentemperatur, 149,2 g (1,08 Mol) Schwefelsäure, 71,0 Gew. % zugepumpt. Die Manteltemperatur wird anschliessend auf ca. 298 K erhöht und das Reaktionsgemisch 7 Stunden bei einer Innentemperatur von 298 ± 0,5 K und einem Druck von (3 x 10⁵) bis (5 x 10⁵) Pa gerührt. Das Reaktionsgemisch drückt man auf eine gerührte Mischung bestehend aus 500 g Eis, 500 g Wasser und 300 ml (3 Mol) Natronlauge, 30 Gew. % ab und spült den Autoklaven anschließend mit 200 - 300 ml Petrolether, der ebenfalls zum Eis-, Wasser- / Natronlauge-Gemisch gegeben wird. Das Gemisch wird auf Raumtemperatur erwärmt und die organische Phase abgetrennt. Die wässerige Phase wird noch dreimal mit jeweils 100 - 150 ml Petrolether extrahiert und die organischen Phasen anschliessend vereinigt. Am Rotationsverdampfer werden die organischen Phasen bei einer Badtemperatur von 323 K und (3 x 10³) Pa (Endwert) eingeengt. Zur Reinigung des Rohproduktes wird dieses, unter Zusatz von ca. 0,5 g Magnesiumoxyd, über eine 30 cm Füllkörperkolonne (Raschigringe 5 x 5 mm) im Vakuum über Kopf destilliert. Man erhält 360,7 g (87,0 % der Theorie) Bernsteinsäure-di-tert.-butylester (Kp: 0,8 - 0,9 Pa bei 321 bis 324 K) mit einer gaschromatographisch ermittelten Reinheit von 99,4 % (Flächenprozent) entsprechend 86,5 % der Theorie à 100 %.

## Patentansprüche

1. Verfahren zur Herstellung von Bernsteinsäure-di-tert.-butylester der Formel 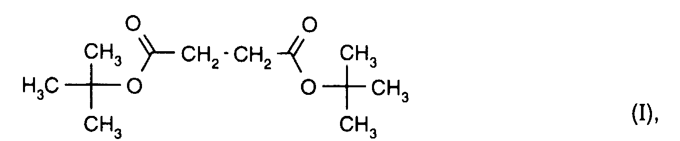 durch protonenkatalysierte Veresterung von Bernsteinsäure mit Isobuten, dadurch gekennzeichnet, dass man man Bernsteinsäure oder Bernsteinsäureanhydrid oder Mischungen davon mit Isobuten in Gegenwart eines wasserhaltigen, sauren Veresterungskatalysators, umsetzt, mit der Massgabe, dass man keine Schwefelsäure in einer Konzentration höher als 95 Gew.% einsetzt.

2. Verfahren nach Anspruch 1, dadurch gekennzeichnet, daß das Molverhältnis von Bernsteinsäure oder Bernsteinsäureanhydrid oder Mischungen davon zu Isobuten 1:2 bis 1: 50 beträgt.

3. Verfahren nach Anspruch 1, dadurch gekennzeichnet, daß das Molverhältnis von Bernsteinsäure zu Bernsteinsäureanhydrid in der Mischung im Bereich von 1:100 bis 100:1 liegt.

4. Verfahren nach Anspruch 1, dadurch gekennzeichnet, daß man als saure Veresterungskatalysatoren 40 bis 90 Gew. %-ige wässerige Schwefel- oder Phosphorsäure oder organische Sulfonsäuren, wie beispielsweise Methansulfonsäure verwendet.

5. Verfahren nach Anspruch 1, dadurch gekennzeichnet, daß man bei Verwendung von Bernsteinsäure 65 bis 75 Gew. %-ige und bei Verwendung von Bernsteinsäureanhydrid 55 bis 65 Gew. %-ige wässerige Schwefelsäure verwendet.

6. Verfahren nach Anspruch 1, dadurch gekennzeichnet, daß man pro Mol Bernsteinsäure oder Bernsteinsäureanhydrid oder einer Mischungen davon 0,1 bis 1,3 Mol Veresterungskatalysator einsetzt.

7. Verfahren nach Anspruch 1, dadurch gekennzeichnet, daß man die Veresterung in einem Temperaturbereich von 263 bis 333 K und einem Druck von (1 x 10⁵) bis (25 x 10⁵) Pa durchführt.

8. Verwendung des nach dem Verfahren gemäß Anspruch 1 hergestellten Bernsteinsäuredi-tert.-butylesters der Formel (I), zur Herstellung von 1,4-Diketopyrrolo-[3,4-c]-pyrrol-Pigmenten.
